# EUROPEAN PATENT APPLICATION

(11) **EP 1 595 531 A1**
(43) Date of publication of application: **16.11.2005**
(21) Application number: 04011424.1
(22) Date of filing: 13.05.2004
(51) Int. Cl.: A61K 9/12, A61K 31/4704, A61M 15/00, B65D 83/14

(54) **Stable pharmaceutical solution formulations for pressurized metered dose inhalers**

(71) Applicant: CHIESI FARMACEUTICI S.p.A., I-43100 Parma (IT)
(72) Inventor: Lewis, David c/o Chiesi Farmaceutici S.p.A., 43100 Parma (IT); Meakin, Brian c/o Chiesi Farmaceutici S.p.A., 43100 Parma (IT); Delcanale Maurizio c/o Chiesi Farmaceutici S.p.A., 43100 Parma (IT); Pivetti, Fausto c/o Chiesi Farmaceutici S.p.A., 43100 Parma (IT); Ganderton, David. c/o Chiesi Farmaceutici S.p.A., 43100 Parma (IT)
(74) Representative: Adam, Holger

(57) **Abstract**

Aerosol solution compositions for use in an aerosol inhaler which comprise 8-hydroxy-5-[(1R)-1-hydroxy-2-[[(1R)-2-(4-methoxyphenyl)-1-methylethyl]amino]ethyl]-2(1H)-quinolinone or a salt thereof, in particular the hydrochloride salt (TA 2005), as an active material, a propellant containing a hydrofluoroalkane, and a cosolvent stabilized by addition of a specific small amount of a high concentrated phosphoric acid and optionally by the use of a suitable can having part or all of its internal metallic surfaces lined with an inert organic coating.

## Description

### BACKGROUND OF THE INVENTION

### FIELD OF THE INVENTION

The present invention relates to stable pharmaceutical solutions to be used with pressurized metered dose inhalers (MDIs) suitable for aerosol administration. In particular, the present invention relates to solutions to be used with pressurized metered dose inhalers (MDIs), which are suitable for aerosol administration containing the β₂-agonist 8-hydroxy-5-[(1R)-1-hydroxy-2-[[(1R)-2-(4-methoxyphenyl)-1-methylethyl]amino]ethyl]-2(1H)-quinolinone or a salt thereof (the hydrochloride salt is hereinafter referred to as TA 2005) and which is stable at room temperature for a pharmaceutically acceptable shelf-life.

### DISCUSSION OF THE BACKGROUND

Pressurized metered dose inhalers are well known devices for administering pharmaceutical products to the respiratory tract by inhalation.

Drugs commonly delivered by inhalation include bronchodilators such as β₂-agonists and anticholinergics, corticosteroids, anti-leukotrienes, anti-allergies and other materials that may be efficiently administered by inhalation, thus increasing the therapeutic efficacy and reducing side effects.

MDIs use a propellant to expel droplets containing the pharmaceutical product to the respiratory tract as an aerosol. Formulations for aerosol administration *via* MDIs can be solutions or suspensions. Solution formulations offer the advantage of being homogeneous with the active ingredient and excipients completely dissolved in the propellant vehicle or its mixture with suitable co-solvents such as ethanol. Solution formulations also obviate physical stability problems associated with suspension formulations so assuring more consistent uniform dosage administration.

For many years the preferred propellants used in aerosols for pharmaceutical use have been a group of chlorofluorocarbons which are commonly called Freons or CFCs, such as CCl₃F (Freon 11 or CFC-11), CCl₂F₂ (Freon 12 or CFC-12), and CClF₂-CClF₂ (Freon 114 or CFC-114).

Recently, the chlorofluorocarbon (CFC) propellants such as Freon 11 and Freon 12 have been implicated in the destruction of the ozone layer and their production is being phased out.

Hydrofluoroalkanes ((HFAs) known also as hydro-fluoro-carbons (HFCs)) contain no chlorine and are considered less destructive to ozone and these are proposed as substitutes for CFCs.

HFAs and in particular 1,1,1,2-tetrafluoroethane (HFA 134a) and 1,1,1,2,3,3,3-heptafluoropropane (HFA 227) have been acknowledged to be the best candidates for non-CFC propellants and a number of medicinal aerosol formulations using such HFA propellant systems have been disclosed.

Due to the higher polarity of the HFA propellants, in particular of HFA 134a (dielectric constant D ≥ 9.5), with respect to CFC vehicles (D ≤ 2.3), HFA solution formulations may suffer to a greater extent of chemical stability problems with respect to the corresponding CFC formulations.

Preparation of stable HFA solution formulations is even more critical when bronchodilator β₂-agonists belonging to the class of the phenylalkylamino derivatives are concerned; in particular, 8-hydroxy-5-[(1R)-1-hydroxy-2-[[(1R)-2-(4-methoxyphenyl)-1-methylethyl] amino]ethyl]-2(1H)-quinolinone suffers from chemical stability problems in this kind of vehicle and it is highly susceptible to chemical degradation.

Moreover, 8-hydroxy- 5-[(1R)-1-hydroxy-2-[[(1R)-2-(4-methoxyphenyl)-1-methylethyl]amino]ethyl]-2(1H)-quinolinone is highly potent and its dosage-strength level is considerably less than many other drugs which can be administered by MDIs. Thus, its concentration in the aerosol formulation is very low and this factor, together with its chemico-physical properties, increases the difficulties in preparing a formulation which is stable and provides good dosage reproducibility when administered by MDIs.

In consideration of the problems outlined, it would be highly advantageous to provide formulations in the form of HFA solutions to be administered by MDIs aimed at providing pharmaceutical doses of 8-hydroxy-5-[(1R)-1-hydroxy-2-[[(1R)-2-(4-methoxyphenyl)-1-methylethyl]amino]ethyl]-2(1H)-quinolinone and its therapeutic salts, in particular the hydrochloride salt (i.e. TA 2005), which do not require the use of refrigeration, remain chemically and physically stable during storage at ambient conditions of temperature and are characterized by adequate life time.

### SUMMARY OF THE INVENTION

Accordingly, it is one object of the present invention to provide formulations in the form of HFA solutions to be administered by MDIs for providing pharmaceutical doses of 8-hydroxy-5-[(1R)-1-hydroxy-2-[[(1R)-2-(4-methoxyphenyl)-1-methylethyl]amino]ethyl]-2(1H)-quinolinone or its salts into the lower respiratory tract of patients suffering from pulmonary diseases such as asthma, characterized by adequate shelf-life at room temperature.

### DETAILED DESCRIPTION OF THE PREFERRED EMBODIMENTS

According to the present invention there is provided a pharmaceutical composition comprising as active compound 8-hydroxy-5-[(1R)-1-hydroxy-2-[[(1R)-2-(4-methoxyphenyl)-1-methylethyl]amino]ethyl]-2(1H)-quinolinone or a salt thereof, in particular the hydrochloride salt, in a solution of a liquefied HFA propellant, a co-solvent selected from pharmaceutically acceptable alcohols, in which the apparent pH of the solution has been adjusted to between 2.5 and 5.5 by addition of a specific amount of high concentrated (i.e. more than about 10 M, preferably more than about 12 M and in particular about 15 M) phosphoric acid. The composition of the invention may be contained in a pressurized MDI having part or all of its internal metallic surfaces lined with an inert organic coating.

In fact, it has been found that, in the case of 8-hydroxy-5-[(1R)-1-hydroxy-2-[[(1R)-2-(4-methoxyphenyl)-1-methylethyl]amino]ethyl]-2(1H)-quinolinone or its salts the chemical stability in HFA solution formulations could be dramatically improved by a proper and combined selection of the kind of cans as well as the apparent pH range adjusted by the use of high concentrated phosphoric acid in specific amounts. The attribution 'apparent' is used as pH is indeed characteristic of aqueous liquids where water is the dominant component (Mole Fraction > 0.95). In relatively aprotic solvents, such as the HFA-ethanol vehicles used in these studies, protons are non-hydrated; their activity coefficients differ significantly from those in aqueous solution. Although the Nernst equation with respect to EMF applies and the pH-meter glass electrode system will generate a variable milli-volt output according to proton concentration and vehicle polarity, the "pH" meter reading is not a true pH value. The meter reading represents an apparent pH or acidity function (pH').

When 8-hydroxy-5-[(1R)-1-hydroxy-2-[[(1R)-2-(4-methoxyphenyl)-1-methylethyl]amino]ethyl]-2(1H)-quinolinone HCl [TA 2005] in a concentration of 4 µg/50 µl was titrated with 0.08 M hydrochloric acid in a model vehicle system commercially available (HFA 43-10MEE, Vertrel XF, Dupont), according to a method developed by the applicant, the pH' profile exhibited a shallow negative slope to about pH' = 5.0; thereafter the acidity function dropped abruptly.

It has been then found that in presence of lower concentrations of TA 2005 and for example with a concentration of 1 µg/50 µl (0.002 % w/v) in presence of 0.08 M HCl, the pH interval is of 2.5-5.5 and the degree of stabilization is determined by the percent amount of the acid.

Further experiments have shown, and it will be described in detail in the following, that TA 2005 can be better stabilized with high concentrated phosphoric acid, and in particular with 15 M phosphoric acid.

Moreover, it has been noticed that the stabilizing effect of phosphoric acid is not strictly correlated with its w/w percent amount in the formulation and it is present in a concentration interval from 0.001 to 0.040 % by weight on the total weight of the formulation.

The corresponding apparent pH interval is of 2.5-5.5, preferably 3.0-5.5, more preferably 3.5-5.0.

On the other hand, it has been observed that the use of inert containers, in particular having part or all of their internal metallic surfaces lined with an inert organic coating can enhance the chemical stability of the active ingredient in the HFA propellant solution.

According to a particular embodiment of the invention, there is provided a pressurised MDI for administering pharmaceutical doses consisting of a container lined with an inert coating, filled with a pharmaceutical composition comprising a solution of 8-hydroxy-5-[(1R)-1-hydroxy-2-[[(1R)-2-(4-methoxyphenyl)-1-methylethyl]amino]ethyl]-2(1H)-quinolinone in HFA 134a as a propellant in turn containing from about 8 to about 15% w/w ethanol as a co-solvent, the apparent pH of said solution having been adjusted to between 3.0 and 5.5 by addition of 0.009 % w/w of 15 M phosphoric acid. The expression '% w/w' means the weight percentage of the component with respect to the total weight of the composition.

The active ingredient in such a composition filled in said container has a good chemical stability and shelf-life at room temperature and meets the requirements of the ICH Guideline Q1A referring to "Stability Testing of new Active Substances (and Medicinal Products)", wherein a significant change for a drug product is defined as a 5% change in assay from its initial value.

The pharmaceutical composition of the invention may further contain a low volatility component in order to increase the mass median aerodynamic diameter (MMAD) of the aerosol particles on actuation of the inhaler.

In WO 98/34596, the applicant described solution compositions for use in an aerosol inhaler, comprising an active material, a propellant containing a hydrofluoroalkane (HFA), a cosolvent and further comprising a low volatility component to increase the mass median aerodynamic diameter (MMAD) of the aerosol particles on actuation of the inhaler. Said application does not address the technical problem of the chemical stability of the active ingredient but it rather concerns the drug delivery to lungs.

In International Application No. PCT/EP99/09002 filed on 23/11/99 published on June 2, 2000, as WO 00/30608 ('608), the applicant has disclosed pressurized MDIs for dispensing a solution of an active ingredient in a hydrofluorocarbon propellant, a co-solvent and optionally a low-volatility component characterized in that part or all of the internal surfaces of said inhalers consist of stainless steel, anodised aluminum or are lined with an inert organic coating. The '608 application makes no mention of a critical role of a mineral acid and in particular of phosphoric acid in order to improve the chemical stability of the active ingredient in the composition. On the contrary, it states that ipratropium bromide (one of the possible active ingredients) is stable in particular kinds of cans, with or without acids.

EP 673240 proposes the use of acids as stabilizers for preventing the chemical degradation of the active ingredient in aerosol solution formulations comprising HFAs. Most examples relate to ipratropium bromide, an anticholinergic drug and only an example is presented for a β₂-agonist, *i.e.,* fenoterol. Although salbutamol is claimed, no exemplary formulations are provided. The stability data are reported only for ipratropium bromide and no difference is made between the use of organic and inorganic acids. Phosphoric acid is only cited among a list of possible inorganic acids. Furthermore, apart from ipratropium bromide, in EP 673240 no guidance is given with respect to the amount of acid which has to be added in order to stabilize the medicaments without compromising the stability of the whole composition in the can. The only hint can be found on page 5, lines 15 to 16, which says that an amount of inorganic acid should be added to obtain a pH value from 1 to 7, so a very broad and generic range.

WO 98/34596 refers to solution formulations containing a propellant and a physiologically acceptable polymer which could help the solubilization and the stability as well of the active ingredients.

WO 00/06121 refers to propellant mixtures for aerosol dinitrogen monoxide and a hydrofluoroalkane in the preparation of suspension and solution aerosols. The use of dinitrogen monoxide may improve the stability on storage of oxidation-sensitive active ingredients. As far as β₂-agonists such as levosalbutamol sulfate, formoterol fumarate and salmeterol xinafoate, only examples referred to suspensions are reported.

In EP 1 157 689 ('689) of the applicant, stability data of a HFA 134a solution formulation containing 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) 3.5 µg/50 µl dose, 12% w/w ethanol, 1% w/w isopropyl myristate stabilised by different amounts of HCl 0.08M (1.0 and 1.4 µl) were reported (Example 7).

The formulations seemed to be provided with quite good stability. Nevertheless, when the present inventors repeated the test, they noticed a progressive degradation of the active ingredient in the formulation.

Moreover, the formulation exemplified in '689 contained isopropyl myristate as a low volatility compound in order to increase the MMAD (mass median aerodynamic diameter) of the delivered particles. It has been subsequently found that it would be highly advantageous to provide highly efficient TA 2005 formulations characterised by a deeper lung penetration by virtue of a significant fraction, of at least 30%, of fine particles, with a diameter equal or less than 1.1 µm. Therefore the low volatility compound should be avoided.

It has been subsequently also found that in this kind of highly efficient formulations, characterized by the presence of a fraction of particles equal to or less than 1.1 µm higher than 30% and even than 50% or more, TA 2005 can be present in a very low concentration, starting from 0.0005% w/v based on the total volume of the composition.

Said compositions have been described in another previous application of the applicant, WO 03/074025 ('025), wherein stability data of a HFA solution formulation comprising 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) stabilized by HCl were reported.

The stability was determined on a formulation delivering 4 µg of active compound per actuation, stored upright at 5° C: in said refrigerated conditions, after nine months, the TA 2005 assay was higher than 95%.

However, it has then been found by the present inventors that when present in lower concentrations and in other storage conditions, the active ingredient in the formulation rapidly degrades.

On the other hand, refrigeration is undesirable because many patients are required to carry the aerosol canisters on their persons.

According to the first aspect of the present invention, it has been found by the present inventors that, while according to the previous disclosure of '025 the preferred mineral acid was hydrochloric acid, the chemical stability of 8-hydroxy-5-[(1R)-1-hydroxy-2-[[(1R)-2-(4-methoxyphenyl)-1-methylethyl]amino]ethyl]-2(1H)-quinolinone and its salts is enhanced by small amounts of high concentrated phoshoric acid, preferably 15M phosphoric acid, comprised between 0.002 and 0.027% w/w in the formulation. The mineral acid that better stabilizes the active ingredient 8-hydroxy-5-[(1R)-1-hydroxy-2-[[(1R)-2-(4-methoxyphenyl)-1-methylethyl]amino]ethyl]-2(1H)-quinolinone and its salts in the formulation is phosphoric acid.

The aerosol formulations containing phosphoric acid are surprisingly stable at room temperature for a long life time.

According to a further aspect of the present invention there is provided a method of filling an aerosol inhaler with a composition of the invention, the method comprising:
(a) preparing a solution of 8-hydroxy-5-[(1R)-1-hydroxy-2-[[(1R)-2-(4-methoxyphenyl)-1-methylethyl]amino]ethyl]-2(1H)-quinolinone or its salts in one or more co-solvents optionally containing an appropriate amount of a low volatility component;
(b) filling the device with said solution;
(c) adding a pre-determined amount of a phosphoric acid;
(d) adding a propellant containing a hydrofluoroalkane (HFA); and
(e) crimping with valves and gassing.

The active ingredient used in the aerosol compositions of the present invention is a long-acting β₂-adrenergic agonist and its combinations with other active ingredients and in particular a corticosteroid or an antimuscarinic drug. Examples of a corticosteroid are: beclomethasone dipropionate, fluticasone propionate, butixocort, mometasone furoate, triamcinolone acetonide, budesonide and its 22R-epimer, ciclesonide and rofleponide. Examples of antimuscarinic are ipratropium bromide, oxitropium bromide and tiotropium bromide.
The active ingredient is 8-hydroxy-5-[(1R)-1-hydroxy-2-[[(1R)-2-(4-methoxy-phenyl)-1-methylethyl]amino]ethyl]-2(1H)-quinolinone or a salt thereof. The preferred salt is the hydrochloride salt, sometimes referred to as TA 2005.

Although the preferred formulations of the invention are in the form of solutions, in case of the combinations, one of the two active ingredients could be present in suspension.

TA 2005 may be prepared as described in U.S. Patent No. RE 33,024.

We prefer the formulation to be suitable for delivering a therapeutic amount of the active ingredient in one or two actuations. Preferably the formulation will be suitable for delivering 0.5-6 µg/dose, more preferably 1-4 µg/dose and in particular 1 to 2 µg/dose, either alone or in combination. By "dose" we mean the amount of active ingredient delivered by a single actuation of the inhaler.

The formulations of the present invention will be preferably contained in cans having part or all of the internal surfaces lined with an inert organic coating. Examples of preferred coatings are epoxy-phenol resins, perfluoroalkoxyalkane, perfluoroalkoxyalkylene, perfluoroalkylenes such as polytetrafluoroethylene, fluorinated-ethylene-propylene, polyether sulfone and copolymers of fluorinated-ethylene-propylene polyether sulfone. Other suitable coatings could be polyamide, polyimide, polyamideimide, polyphenylene sulfide or their combinations.

The most preferred coatings are perfluoroalkoxyalkane, perfluoroalkoxy-alkylene, perfluoroalkylenes such as polytetrafluoroethylene and fluorinated-ethylene-propylene.

To further improve the stability, cans having a rolled-in rim and preferably a part or full rollover rim can be used.

The formulation is actuated by a metering valve capable of delivering a volume of between 50 µl and 100 µl.

Metering valves fitted with gaskets made of butyl rubber, in particular bromobutyl rubber of the kind described in WO 03/078538 are preferred in order to further improve the stability of the active ingredient in the formulation.

The hydrofluorocarbon propellant is preferably selected from the group of HFA 134a, HFA 227 and mixtures thereof.

The co-solvent is usually an alcohol, preferably ethanol.

The apparent pH range is advantageously comprised between 2.5 and 5.5, preferably between 3.0 and 5.5 , even more preferably between 3.5 and 5.0. Rather concentrated, i.e. more than about 10 M, preferably more than about 12 M and most preferably about 15 M phosphoric acid is used to adjust the apparent pH.

The amount of acid to be added to reach the desired apparent pH will be pre-determined in the model vehicle reported before.

The active ingredient 8-hydroxy-5-[(1R)-1-hydroxy-2-[[(1R)-2-(4-methoxyphenyl)-1-methylethyl]amino]ethyl]-2(1H)-quinolinone or its salt is stabilized with rather concentrated, preferably 15 M phosphoric acid. In particular, it is preferred to add phosphoric acid in an amount equivalent to 0.001 to 0.040% w/w of 15M phosphoric acid, based on the total weight of the composition, preferably 0.001 to 0.030% w/w of 15M phosphoric acid, based on the total weight of the composition, more preferably 0.002 to 0.027% w/w of 15M phosphoric acid, based on the total weight of the composition. For the purposes of the invention a still high concentrated phosphoric acid other than 15 M can be utilized. In this case, the person skilled in the art will be able to determine the right percent amount in view of the disclosure in the present application. In this embodiment it may also be preferred to avoid addition of the low-volatility component to increase the fraction of particles with a diameter less than or equal to 1.1 µm to at least 30% and to provide deeper lung penetration.
The 8-hydroxy-5-[(1R)-1-hydroxy-2-[[(1R)-2-(4-methoxyphenyl)-1-methylethyl]amino]-ethyl]-2(1H)-quinolinone concentration will vary between 0.0005 % and 0.024 % w/v, based on the total volume of the composition, in order to deliver 0.5-6 µg per actuation, preferably between 0.001 % and 0.016 % w/v, based on the total volume of the composition, in order to deliver 1 to 4 µg per actuation, more preferably between 0.001 % and 0.008 % w/v, based on the total volume of the composition, in order to deliver 1 to 2 µg per actuation. For instance, for 1 and 2 µg/dose, wherein a 63 µl metering volume is used, the final concentrations of the hydrochloride salt TA 2005 delivered per actuation would be 0.0016 % and 0.0032 % w/v, respectively, based on the total volume of the composition. The amount of co-solvent in the composition is suitably 6 to 30 % w/w, preferably 6 to 25 % w/w, more preferably 6 to 20 % w/w, even more preferably 8 to 15 % w/w, based on the total weight of the composition.

In these conditions, the stability of TA 2005 is enhanced also at the very low dosage strength of 1 µg per actuation.

The apparent pH values are preferably comprised between 3.0 and 5.0.

Other features of the invention will become apparent in the course of the following descriptions of exemplary embodiments which are given for illustration of the invention and are not intended to be limiting thereof.

### EXAMPLES

In the following examples and comparative examples, and throughout this specification, all parts and percentages are by weight, and all temperatures are in degrees Celsius, unless expressly stated to be otherwise.

### Comparative Example 1 (corresponding to Example 7 of EP 1 157 689)

Stability of acidified 8-hydroxy-5-[(1R)-1-hydroxy-2-[[(1R)-2-(4-methoxyphenyl)-1-methylethyl]amino]ethyl]-2(1H)-quinolinone hydrochloride (TA 2005)-HFA 134a solutions in cans coated with a fluorocarbon polymer.

A formulation of 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) (3.5 µg/50 µl) was prepared by dissolving 0.84 mg of the active ingredient in HFA 134a containing 12% w/w ethanol and 1.0% w/w of ispropyl myristate. pMDI coated cans containing 1.0 and 1.4 µl of 0.08 M hydrochloric acid (corresponding respectively to an apparent pH of about 4.8 and 3.2) were set down on storage, upright at 50°C, and samples taken for analysis of TA 2005 contents at appropriate intervals.

Stability data obtained are given in Table 1.

Each value is expressed as per cent nominal drug concentration.

The results indicate that formulations containing from 1.0 to 1.4 µl of 0.08 M HCl, whose apparent pH is comprised between 3.0 and 5.0, are stable for almost three months at 50° C.

**Table 1:**

| 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) formulations of Comparative Example 1 - Stability data at 50°C | | | |
|---|---|---|---|
| 0.08M HCl µl per can | Storage Condition | | |
| | Initial | 50°C; 22 days upright | 50°C; 83 days upright |
| 1.0 | 100.0 | 98.3 | 99.4 |
| 1.4 | 100.0 | 98.2 | 98.8 |

### Comparative Example 2 (corresponding to Example 1 of WO 03/074025)

A formulation for delivering a nominal dose of 1 µg of active ingredient per actuation, was prepared with the following formulation shown in Table 2.

**Table 2:**

| *Components* | *Amounts* | | |
|---|---|---|---|
| | Per unit | | Nominal dose |
| | mg | % | µg |
| TA 2005 | 0.15 | 0.0016 w/v | 1 |
| Ethanol | 1650 | 15 w/v | - |
| HCl 0.1 M | 2.0 * | 0.018 w/w | - |
| HFA 134a q.s. to 9.45 ml | 9347.85 | - | - |

| | | | |
|---|---|---|---|
| * equivalent to 2.0 µl | | | |

The formulation (120 actuations/canister, overage of 30 actuations) was filled in aluminium canisters having the internal surface coated with Teflon and fitted with a metering valve having a 63 µl metering chamber. An actuator with an orifice diameter of 0.22 mm was used.

Analogous formulations able of delivering a nominal dose of 2, 3 or 4 µg per actuation of active ingredient were prepared. The 1 µg per dose formulation has been used to determine the aerodynamic particle size distribution.

A stability study on a formulation able to deliver 4 µg per actuation was initiated storing the cans upright at 5° C.

Results were obtained as a mean of 2 cans.

After nine months the 8-hydroxy-5-[(1R)-1-hydroxy-2-[[(1R)-2-(4-methoxy-phenyl)-1-methylethyl] amino]ethyl]-2(1H)-quinolinone hydrochloride assay was higher than 95% and therefore met with the requirements of the ICH guideline Q1A referring to "Stability Testing of new Active Substances (and Medicinal Products)".

### Comparative Example 3:

Two aluminum canisters having the internal surface coated with teflon and fitted with commercial valves having a 63 µl metering chamber were filled with the following formulations shown in Table 3.

**Table 3:**

| Components | Amounts per unit | | | |
|---|---|---|---|---|
| | mg | % | mg | % |
| 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) (1µg/63µl) | 0.154 | 0.0016 w/v | 0.154 | 0.0016 w/v |
| Ethanol | 1650.0 | 15 w/w | 1650.0 | 15 w/w |
| Hydrochloric acid 0.1M | 2.00 | 0.018 w/w | 3.00 | 0.027 w/w |
| HFA 134a q.s. to 9.45 ml | 9347.85 | - | 9346.85 | - |

A stability study was carried out by storing the formulations in upright cans at 40° C and 75% relative humidity. After three months of storage under these conditions the percent amount of TA 2005 was 73% and 77%, respectively.

According to the results of Comparative Examples 1 to 3, TA 2005 (8-hydroxy-5-[(1R)-1-hydroxy-2-[[(1R)-2-(4-methoxyphenyl)-1-methylethyl]amino]ethyl]-2(1H)-quinolinone hydrochloride) can be stabilized by the use of hydrochloric acid if TA 2005 is present in the solution formulation in a comparatively high concentration (3.5 µg/50 µl; 4 µg /63 µl, respectively) and in refrigerated conditions. However, if it is present in a rather low concentration as desired for this kind of active ingredient (e.g. 1 µg/63 µl), it can no longer be stabilized by the use of hydrochloric acid. The active ingredient 8-hydroxy-5-[(1R)-1-hydroxy-2-[[(R)-2-(4-methoxyphenyl)-1-methylethyl]amino]ethyl]-2(1H)-quinolinone hydrochloride is a very potent long acting β₂-agonist active at a very low dosage-strength, which should be applied in a low concentration. Moreover, the storage in a refrigerator should be avoided.

As it will be demonstrated by the results of the following example, 8-hydroxy-5-[(1R)-1-hydroxy-2-[[(1R)-2-(4-methoxyphenyl)-1-methylethyl]amino]ethyl]-2(1H)-quinolinone hydrochloride can, however, be stabilized also in very low concentrations (e.g. 1 µg/63 µl) by the use of phosphoric acid in an amount equivalent to 0.001 to 0.040 % w/w, preferably 0.001 to 0.030 % w/w, more preferably 0.002 to 0.027 % w/w of 15 M phosphoric acid, based on the total weight of the composition.

### Example:

An analogous formulation (see Table 4) for delivering a nominal dose of 1 µg of 8-hydroxy-5-[(1R)-1-hydroxy-2-[[(1R)-2-(4-methoxyphenyl)-1-methylethyl]amino]ethyl]-2(1H)-quinolinone hydrochloride (TA 2005) per actuation was prepared with the composition as follows, utilizing phosphoric acid as a stabilizer in place of hydrochloric acid.

**Table 4:**

| Components | Amounts per unit | |
|---|---|---|
| | mg | % |
| TA 2005 (1µg/63µl) | 0.154 | 0.0016 w/v |
| Ethanol | 1650.0 | 15.00 w/w |
| Phosphoric acid 15M | 1.00 | 0.009 w/w |
| HFA 134a q.s. to 9.72 ml | 9348.8 | - |

Analogously, formulations capable of delivering a nominal dose of 0.5, 1.5, 2, 2.5, 3, 3.5 or 4 µg of active ingredient per actuation may also be prepared.

The formulation of Table 4 was filled in aluminum canisters having the internal surface coated with teflon and fitted with commercial valves having a 63 µl metering chamber.

A stability study was carried out by storing the formulation in upright cans at 40° C and 75% relative humidity. After three months of storage under these conditions the percent amount of TA 2005 was 98%.

Obviously, numerous modifications and variations of the present invention are possible in light of the above teachings. It is therefore to be understood that, within the scope of the appended claims, the invention may be practiced otherwise than as specifically described herein.

All patents and other references mentioned above are incorporated in full herein by this reference, the same as if set forth at length.

## Claims

1. A composition, which comprises 8-hydroxy-5-[(1R)-1-hydroxy-2-[[(1R)-2-(4-methoxyphenyl)-1-methylethyl]amino]ethyl-2(1H)-quinolinone or a salt thereof, in particular the hydrochloride salt (TA 2005), a liquefied HFA propellant, a co-solvent selected from pharmaceutically acceptable alcohols, and high concentrated phosphoric acid, wherein said composition is in the form of a solution, and said solution has an apparent pH of between 2.5 and 5.5.

2. The composition according to Claim 1, wherein said liquefied HFA propellant is at least one member selected from the group consisting of HFA 134a, HFA 227, and mixtures thereof.

3. The composition according to Claim 1 or 2, wherein said co-solvent is ethanol.

4. The composition according to any one of Claims 1-3, wherein said phosphoric acid is present in an amount equivalent to 0.001 to 0.040% w/w of 15M phosphoric acid, based on the total weight of the composition.

5. The composition according to any one of Claims 1-3, wherein said phosphoric acid is present in an amount equivalent to 0.001 to 0.030% w/w of 15M phosphoric acid, based on the total weight of the composition.

6. The composition according to any one of Claims 1-3, wherein said phosphoric acid is present in an amount equivalent to 0.002 to 0.027% w/w of 15M phosphoric acid, based on the total weight of the composition.

7. The composition according to any one of Claims 1-6, which has an apparent pH of 3.0 to 5.5.

8. The composition according to any one of Claims 1-6, which has an apparent pH of 3.5 to 5.0.

9. The composition according to any one of Claims 1-8, wherein said 8-hydroxy-5-[(1R)-1-hydroxy-2-[[(1R)-2-(4-methoxyphenyl)-1-methylethyl]amino]ethyl]-2(1H)-quinolinone or salt thereof is present in an amount of 0.0005 % to 0.024 % w/v.

10. The composition according to any one of Claims 1-8, wherein said 8-hydroxy-5-[(1R)-1-hydroxy-2-[[(1R)-2-(4-methoxyphenyl)-1-methylethyl]amino]ethyl]-2(1H)-quinolinone or salt thereof is present in an amount of 0.001 % to 0.016 % w/v.

11. The composition according to any one of Claims 1-10, wherein said co-solvent is present in an amount of 6% to 30% w/v.

12. The composition according to any one of Claims 1-10, wherein said co-solvent is present in an amount of 6% to 25% w/v.

13. A pressurized metered dose inhaler, which contains a composition, wherein said composition comprises 8-hydroxy-5-[(1R)-1-hydroxy-2-[[(1R)-2-(4-methoxyphenyl)-1-methylethyl]amino]ethyl]-2(1H)-quinolinone or a salt thereof, in particular the hydrochloride salt (TA 2005), a liquefied HFA propellant, a co-solvent selected from pharmaceutically acceptable alcohols, and high concentrated phosphoric acid, wherein said composition is in the form of a solution, and said solution has an apparent pH of between 2.5 and 5.5.

14. The pressurized metered dose inhaler according to Claim 13, wherein said liquefied HFA propellant is at least one member selected from the group consisting of HFA 134a, HFA 227, and mixtures thereof.

15. The pressurized metered dose inhaler according to Claims 13 or 14, wherein said co-solvent is ethanol.

16. The pressurized metered dose inhaler according to any one of Claims 13-15, wherein said phosphoric acid is present in said composition in an amount equivalent to 0.001 to 0.040% w/w of 15M phosphoric acid, based on the total weight of the composition.

17. The pressurized metered dose inhaler according to any one of Claims 13-15, wherein said phosphoric acid is present in said composition in an amount equivalent to 0.001 to 0.030% w/w of 15M phosphoric acid, based on the total weight of the composition.

18. The pressurized metered dose inhaler according to any one of Claims 13-15, wherein said phosphoric acid is present in said composition in an amount equivalent to 0.002 to 0.027% w/w of 15M phosphoric acid, based on the total weight of the composition.

19. The pressurized metered dose inhaler according to any one of Claims 13-18, wherein said composition has an apparent pH of 3.0 to 5.5.

20. The pressurized metered dose inhaler according to any one of Claims 13-18, wherein said composition has an apparent pH of 3.0 to 5.0.

21. The pressurized metered dose inhaler according to any one of Claims 13-18, wherein said TA-2005 or salt thereof is present in said composition in an amount of 0.0005 % to 0.024 % w/v.

22. The pressurized metered dose inhaler according to any one of Claims 13-18, wherein said 8-hydroxy-5-[(1R)-1-hydroxy-2-[[(1R)-2-(4-methoxyphenyl)-1-methylethyl]amino]ethyl]-2(1H)-quinolinone or salt thereof is present in said composition in an amount of 0.001 % to 0.016 % w/v.

23. The pressurized metered dose inhaler according to any one of Claims 13-22, wherein said co-solvent is present in said composition in an amount of 6% to 30% w/w.

24. The pressurized metered dose inhaler according to any one of Claims 13-22, wherein said co-solvent is present in said composition in an amount of 6% to 25% w/w.

25. The pressurized metered dose inhaler according to any one of Claims 13-24, wherein part or all of its internal metallic surfaces are lined with an inert organic coating.

26. The pressurized metered dose inhaler according to Claim 25, which is lined with an inert organic coating selected from the group consisting of epoxy-phenol resins, perfluoroalkoxyalkanes, perfluoroalkoxyalkylenes, perfluoroalkylenes, polyether sulfones, copolymers of fluorinated-ethylene-propylene polyether sulfone, and mixtures thereof.

27. A method of filling an aerosol inhaler, said method comprising:
(a) preparing a solution of one or more active ingredients in one or more co-solvents;
(b) filling said inhaler with said solution;
(c) adding a pre-determined amount of phosphoric acid to said solution;
(d) adding a propellant comprising a hydrofluoroalkane (HFA) to said solution; and
(e) crimping with valves and gassing,
wherein at least one of said active ingredients is 8-hydroxy-5-[(1R)-1-hydroxy-2-[[(1R)-2-(4-methoxyphenyl)-1-methylethyl]amino]ethyl]-2(1H)-quinolinone or a salt thereof, in particular the hydrochloride salt (TA 2005), present in an amount of 0.0005 % to 0.024 % w/v, preferably 0.001 % to 0.016 % w/v and said phosphoric acid is present in an amount equivalent to 0.001 to 0.040 % w/w, preferably 0.001 to 0.030 % w/w, more preferably 0.002 to 0.027 % w/w of 15 M phosphoric acid, based on the total weight of the final solution (composition).
